Europäisches Patentamt

European Patent Office

Office européen des brevets

(19)

(11) EP 0 911 058 A1

(12) EUROPEAN PATENT APPLICATION

(43) Date of publication:
28.04.1999 Bulletin 1999/17

(51) Int. Cl.$^6$: A61N 1/05, A61N 1/40,
A61N 1/28

(21) Application number: 98203216.1

(22) Date of filing: 25.09.1998

(84) Designated Contracting States:
AT BE CH CY DE DK ES FI FR GB GR IE IT LI LU
MC NL PT SE
Designated Extension States:
AL LT LV MK RO SI

(30) Priority: 21.10.1997 EP 97850143

(71) Applicants:
• AKZO NOBEL N.V.
6800 SB Arnhem (NL)
Designated Contracting States:
AT BE CH CY DE DK ES FI FR GB GR IE IT LI LU
MC NL PT SE

• PERMASCAND AB
S-840 10 Ljungaverk (SE)
Designated Contracting States:
FI SE

(72) Inventors:
• Nilsson, Eva
852 31 Sundsvall (SE)
• Berendson, Jaak
152 31 Södertälje (SE)

(74) Representative:
Jönsson, Christer
Eka Chemicals AB,
Patent Department,
Box 11556
100 61 Stockholm (SE)

(54) Spherical Electrode

(57) The present invention is related to an electrode for destruction of biological tissue, suitably tumour tissue such as cancer. Prior art electrodes suffers from several disadvantages, such as e.g. a non-uniform current distribution, resulting in a oval and unpredictable destruction zone in the tissue. The present invention provides an electrode for destruction of biological tissue, comprising an electrical conductor (2) with a first thickness ($t_1$), provided with electrical insulation (3) having a second thickness ($t_2$), the conductor being connected to a current source at one end and electrically connected to an electrode head (4) at the other end of the conductor, where the electrode head serves for supplying current to the zone of the biological tissue, wherein the electrode head (4) is substantially spherical (5,10) with a diameter (D), where said diameter of the sphere is larger or equal to said thickness ($t_1,t_2$). The advantage with the electrode of the present invention is above all the creation of a substantially symmetrical circular destruction zone.

Fig.1

## Description

The present invention is related to an electrode for destruction of biological tissue, suitably tumour tissue such as cancer. The tip, or head, of the electrode is substantially spherical in order to obtain a symmetrical circular destruction zone.

One way of treatment of biological tissue is electrochemical treatment of cancer (ECT), which is a rather undeveloped method to eliminate biological tissue. Greater understanding of the mechanisms of electrochemical treatment is needed for the method to get recognised. The development of ECT as a clinically acceptable therapy has been hindered by uncertainties regarding the mechanism of tissue destruction and the parameters that are important in the process. To be able to understand the mechanism behind ECT, and to develop an effective dosage method, a Swedish interdisciplinary research group, was formed in 1993.

In electrochemical treatment of cancer (ECT), the biological tissue is treated with a direct current. The anode is preferably placed in the biological tissue and the cathode is placed in a blood vessel or in fresh surrounding tissue. When using platinum electrodes, the main reactions are decomposition of water along with oxidation and reduction of substances dissolved in tissue. Consequently, the evolution of oxygen, as well as acidification and formation of chlorine, take place at the anode:

$$2H_2O \rightleftharpoons O_2 + 4H^+ + 4e^- \tag{1}$$

$$2Cl^- \rightleftharpoons Cl_2 + 2e^- \tag{2}$$

At the cathode, water is decomposed into hydrogen and hydroxyl ions:

$$2H_2O + 2e^- \rightleftharpoons H_2 + 2OH^- \tag{3}$$

The ionic species produced at the anode and cathode are transported to the surrounding tissue, mainly by means of diffusion and migration. pH values down to 1 are usually obtained in the tissue close to the anode and a pH of about 12 can be measured at the cathode. Due to the formation of acid and base hematin, the affected tissue becomes dark brown. During electrolysis, the current density may usually be about $10^{-2}$ - $10^{-1}$ A/cm$^2$ and the voltage between the anode and cathode can be about 5-15 V. The toxic and destroying action of ECT is assumed to be due to a strong acidification of a zone around the anode, chlorination of tissue constituents close to the anode, electrocoagulation and other possible effects caused by the electric field.

The relatively high current density used for ECT-electrodes, is a prerequisite in order to maintain the oxygen evolution and the acidification around the anode. For this reason, the electrode material and geometry are crucial elements in ECT treatment. For instance, electrodes made of stainless steel would not be suitable for maintaining the oxygen evolution and the acidification around the anode. With respect to the geometry, a needle shaped and tapered head of the electrode causes a non-uniform current distribution, resulting in a oval and unpredictable destruction zone in the tissue.

Endocardiac electrodes, constructed for stimulation of the heart or other muscles, cannot be used for destruction of biological tissue. Endocardiac electrodes uses pulsed current with a low current density. Initially, the current density can be about $10^{-3}$ A/cm$^2$, but is quickly transferred to about $10^{-5}$ A/cm$^2$. If the purpose of the endocardial treatment is just to gently stimulate nerves of a certain type of tissue, the current density will be as low as in the range of $10^{-5}$ A/cm$^2$ down to $10^{-7}$ A/cm$^2$. Besides, the shape and the material of endocardiac electrodes are different compared to electrodes for biological tissue destruction. Moreover, mechanical stability of wire electrodes is often poor due to the tapered end and the overall construction, which implies that wire electrodes only can be used a few times.

US-A-4,677,990 relates to an endocardial electrode having a flexible electrical conductor, which can assume arbitrary curvatures or contours.

EP-B1-0038484 discloses an endocardial electrode arrangement for the intracardial stimulation of the heart. The electrode comprises a plurality of loops of soft material and are fastened to the electrode head. The aim is to fix electrode to the heart wall.

EP-A-316995 relates to an electrode for treatment of body tissue. The electrode is formed by a platinum wire which is passed through a tube of insulating material. The head of the electrode is formed by winding the end of the wire.

The aim with the ECT treatment is to get a uniform, symmetrical and reproducible destruction zone. This aim is met by the electrode of the present invention.

According to the present invention, the above mentioned problems have been solved by providing an electrode for destruction of biological tissue, comprising an electrical conductor with a first thickness, provided with electrical insulation having a second thickness, the conductor being connected to a current source at one end and electrically connected to an electrode head at the other end of the conductor, where the electrode head serves for supplying current to the zone of the biological tissue, wherein the electrode head is substantially spherical with a diameter, where said diameter of the sphere is equal to or larger than said first thickness.

The advantage with the electrode of the present invention is above all the creation of a substantially symmetrical circular destruction zone. This is due to the possibility to create a uniform current distribution with the spherical head of the electrode according to the present invention. One great advantage is the predictability of the destruction area. Further, the mechanical stability of the present new electrode is superior to conventional electrodes and this allows for the electrode to be used in many treatments before it has to be discarded. Besides, this new construction of electrodes opens the possibility for a wide range of applicable embodiments.

According to the present invention, the electrode head has perferably the shape of substantially a smooth and regular sphere, globe or ball, with a certain diameter. It is important that the diameter of the sphere is equal to or larger than the thickness of the electrical conductor in order to give a uniform current distribution resulting in a symmetrical destruction zone in the biological tissue. Preferably, the diameter of the sphere is larger than the thickness of the electrical conductor. This is due to the fact that an electrical conductor with a thickness larger than the electrode head, or sphere, could act as a shield for the current at the treatment. However, the outer diameter of the electrical insulation coating of the electrical conductor may be the same thickness as the diameter of the spherical head, but preferably the thickness of the conductor incorporating the insulation coating is less than the diameter of the sphere. The electrical insulation can be fluoroplastics such as PTFE, PVDF, PFA, FEP, Teflon®, or other suitable plastic material, or the similar. The sphere has suitably a diameter in the range of from about 0.1 mm up to about 5 mm, preferably in the range from about 0.5 mm up to about 3 mm and more preferably in the range from about 0.75 mm up to about 1.5 mm. Although the sphere is substantially spherical, the sphere could be slightly flattened at the so called poles. The thickness of the conductor, preferably also incorporating the thickness of the electrode insulation, is smaller than the diameter of the sphere. The thickness of the conductor can be in the range of from about 0.05 mm up to about 4.5 mm but always slightly smaller than diameter of the spherical electrode head. Suitably the thickness of the conductor is in the range of from about 0.2 mm up to about 2.0 mm and preferably in the range of from about 0.5 mm up to about 1 mm.

In contrast to the pulsed current used in conventional endocardial wire electrodes, the electrode of the present invention preferably uses a constant direct current (DC), i.e. a direct current without substantial variation (in contrast to a pulsed current). The current density applied with the electrode of the present invention is suitably in the range of from about $10^{-4}$ A/cm$^2$ up to about 1 A/cm$^2$, preferably in the range of from about $10^{-4}$ A/cm$^2$ up to about $5 \cdot 10^{-1}$ A/cm$^2$ and more preferably in the range of from about $10^{-3}$ A/cm$^2$ up to about $5 \cdot 10^{-1}$ A/cm$^2$. The electrode according to the present invention can be connected to a DC source so it serves as an anode. Consequently, the electrode connected to a DC source, can operate with a current density in the range of from about $10^{-4}$ A/cm$^2$ up to about $5 \cdot 10^{-1}$ A/cm$^2$.

The total length of the electrode is suitably less than about 500 mm, but suitably exceeding about 100 mm. The length is preferably in the range from about 150 mm up to about 250 mm. At the opposite end of the electrode compared to the electrode head, the electrode is connected to a current source (power supply).

When the electrode is acting as an anode, one or more noble metals alone or in any mixture can be used. Use is suitably made of titanium, tantalum, zirconium or niob, as base material for the anode, but then provided with a coating. Preferably rhodium, palladium, platinum or iridium, or mixtures thereof, is used as material for the anode, as base material, but also as the sole material. The surface of the anode can be coated with a layer of an electrocatalytic active and/or selective material. Suitable materials for the layer are for example, noble metal oxides or platinum, but also other oxides such as e.g. tin oxide are of interest depending on the desired reaction on the anode. As examples of such coating on titanium, mention is made of precious metal oxides or mixtures thereof which are stated in Swedish Patents 345,396, 345,970 and 349,952, or other coatings based on oxides or metals. Suitable coatings on a titanium anode can be Pt, $TiO_2+RuO_2$, $TiO_2+IrO_2$, or mixtures thereof, or other noble metal oxides. In addition, the anode can be coated with graphite, diamond or other higher coal compounds in various structures. With respect to e.g. diamond, the coating can be applied by the CVD-method (Chemical Vapour Deposition) and doped with bor or other suitable materials. Also magnetite ($Fe_3O_4$) could be possible as anode coating.

The spherical head, or the electrical conductor, or even the whole anode may be provided with the aforementioned materials. Suitably only the spherical head of the anode can be made of coated titanium, platinum, rhodium, palladium or noble metal oxides, while the electrical conductor can be made of titanium solely. Preferably the electrical conductor is made of titanium and a spherical head of the anode made of platinum, is welded to the titanium conductor.

When the electrode is acting as a cathode, platinum, iridium or alloys of them, are suitable materials for the cathode.

According to an embodiment of the present invention the spherical head of the electrode can be a separate part of the electrode, which thus can be exchangeable. A spherical head of an electrode may be welded to the end of an electrical conductor. Suitably the sphere is provided with a threaded socket, which implies that the sphere easily can be exchanged. In this way, sphere's can be made in various materials, or coated with various alloys, and be used for different selective treatments of biological tissue in order to reach a desired effect.

In another embodiment, the electrode can be provided with internal channels for supplying salt solution to the area of the tissue which are to be treated. The electrical conductor and the spherical end of the electrode, can e.g. be provided with one or more internal channels. The sphere is suitably provided with several channels, which emerge on evenly spreaded spots around the surface of the sphere. The salt solution can be a physiological saline solution.

According to yet another embodiment, the spherical head of the electrode may comprise of several thin threads which are forced to form a pseudo-spherical head. Such thin threads can initially be arranged parallel to the extension of the electrical conductor and each end of the threads respectively, can be secured to the outmost end of the conductor, i.e. the tip of the electrode. The other ends of said threads are free to move relative the body of the electrical conductor. Consequently, the movable free ends of the threads can be attached e.g. to the bottom of an outer tubing or the similar, surrounding the electrical conductor and arranged a short distance from the tip of the electrical conductor. Said outer tubing can be affected by some means in the direction of the tip of the electrical conductor, where said thin threads are firmly secured, leading to the formation of a spherical head erected by several thin threads. In this way, a spheroid or ellipsoid electro-geometry can be achieved. When the electrode initially is stuck in the body, the thin threads are preferably arranged along the electrode conductor according to the embodiment of the invention, which may be advantageous in order to get the smallest possible influence of the body tissue. When the electrode is fixed in the desired area to be treated, said outer tubing is influenced to affect the threads to form a shape of a spherical head. Suitably this so-called pseudo-sphere may comprise of at least from about 2 threads, more suitably from about 4 threads and preferably from about 8 up to about 16 threads. The thin threads have a thickness smaller than the electrical conductor.

The present invention also relates to a method for carrying out a medical treatment of biological tissue, suitably a tumour, with an ECT-electrode as disclosed. Thus, use is made of an electrode, having a substantially spherical electrode head with a diameter, which is larger or equal to the thickness of the electrical conductor of said electrode. According to the method, at least one first electrode comprising an electrode head (4) and an electrical conductor (2) is inserted in a body and fixed in a desired area of said tissue, at least one second electrode electrically connected to said first electrode, said electrical conductor (2) of the first electrode with a first thickness ($t_1$), provided with an electrical insulation (3) having a second thickness ($t_2$), said conductor being connected to a current source at one end and electrically connected to the electrode head (4) at the other end of the conductor, where the electrode head serves for supplying current to the zone of the biological tissue, where the electrode head (4) is substantially spherical (5,10) with a diameter (D), where said diameter of the sphere is equal to or larger than said first thickness ($t_1$). Said first electrode can serve as anode or cathode. Said second electrode is preferably inserted and fixed at a suitable distance from the anode. A direct current density, suitably in the range of from about $10^{-4}$ A/cm$^2$ up to about 1 A/cm$^2$, is preferably passed between the electrodes by means of a constant-current power supply. The treatment time is suitably chosen with respect to the desired result and specific tissue.

The invention will now be described in more detail with reference to the accompanying drawings. However, the invention is not restricted to the embodiments illustrated, but many other variants are feasible within the scope of the claims.

A preferred embodiment of the invention will be described below with reference to the accompanying drawing in which: Figure 1 illustrates in cross-section an electrode with a spherical head for treatment of biological tissue according to the present invention. Figure 2 demonstrates a side view of a spherical exchangeable electrode head according to an embodiment of the present invention. Figure 3 shows a wire electrode (3a) and a spherical electrode (3b), which have been compared in the example below.

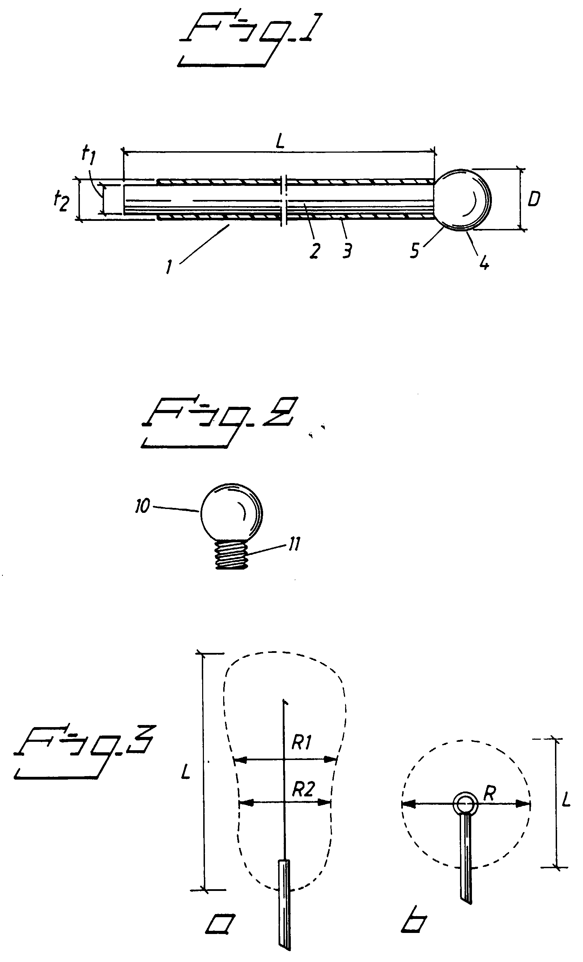

Figure 1 shows according to the present invention an electrode (1) for destruction of biological tissue, comprising an electrical conductor (2), having a thickness ($t_1$), the electrode being provided with electrical insulation (3), having a thickness ($t_2$). The electrical conductor being connected to a current source at one end and electrically connected to an electrode head (4) at the other end of the conductor. The electrode head (4) has the shape of a sphere (5) with a diameter (D). Said diameter of the sphere is larger than said thickness ($t_1$).

Figure 2 illustrates an ECT-electrode according to an embodiment of the present invention with a spherical head (10), provided with a threaded socket (11). The sphere can be unscrewed and exchanged to another spherical head, possibly comprising a different material or design.

Example

All procedures to be described complied with the "Djurförsöksetiska Nämndens" of Sweden (a governmental institution), decision: C112/96. Four female adult CD rats bred by Charles River, Sweden, at a weight of about 350 g were used in the experiment. Anaesthesia was administered by injection of Dormicum®/Hypnorm® intraperitoneally. Two electrode geometries (see figure 3), wire (fig.3a) and sphere (fig.3b), both made by Pt:Ir (9:1), were used in the experiment. Two rats were treated using wire electrodes and two rats were treated using spherical electrodes. The active part of the wire electrodes (fig 3a) had a length of 15 mm and a diameter of 0.2 mm. The rest of the wire surface was insulated by using Teflon®. 1 mm hooks were made in the tips of the electrodes in order to fix the electrodes in the tissue. The spherical electrodes (fig.3b) had a diameter of 1 mm. The spheres were merged to Pt:Ir (9:1) sticks with a diameter of 0.5 mm. The sticks were electrically insulated using Teflon®. The anode was fixed in the fourth mammary gland on the right side (dx 4), guided by a permanent catheter. The cathode was fixed in the same manner in the fourth mammary gland on the left side (sin 4). The electrodes were placed in parallel with the length of the rat. The distance between the electrodes was varied between 52-65 mm. A direct current of $5 \cdot 10^{-3}$ A was passed between the electrodes by means of a constant-current power supply. Linear current ramps were used at the start of the treatment and at the end to avoid muscle twitching. The length of these ramps were 2 minutes. The total treatment time was 52 minutes and consequently the total charge passed between the electrodes was 15 Coulomb. The voltage between the anode and cathode was continuously monitored and was normally about 6-10 V. After the electrolysis the rat was eustanised by injection of pentothal natrium® intra cardially. The macroscopic dark brown coloured destruction zones were then measured. The anodic and cathodic destruction zones were measured from above, through the skin (as shown in Fig. 3). The measured radiuses R1 and R2 were situated 5 mm from the respective tip of the wire electrode. The characteristic dimensions, R1, R2 and L in the wire case (fig.3a), and R and L in the spherical case (fig.3b), of the anodic and cathodic destruction zones are listed in Tables 1 and 2 below. It has to be emphasized that the mammary tissue in rat is not spherical but quite flat with a depth of about 5 mm. Therefore, the destruction zone is also shaped more like a disc with about the same depth.

Table 1

| Rat No. | Size of anodic destruction zone [mm] | | | Size of cathodic destruction zone [mm] | | |
|---|---|---|---|---|---|---|
| | R1 | R2 | L | R1 | R2 | L |
| 1 | 8 | 6 | 18 | 6 | 9 | 17 |
| 2 | 6 | 6 | 19 | 6 | 12 | 22 |

Table 1 shows measured sizes of the macroscopic destruction zone, using wire electrodes. As evident from the results presented, the radiuses (R1 and R2) are about half, to one third, of the length (L) of the destruction zone, which then could be characterised as oval shaped. Besides, the oval is unevenly, irregular shaped.

Table 2

| Rat No. | Size of anodic destruction zone [mm] | | Size of cathodic destruction zone [mm] | |
|---|---|---|---|---|
| | R | L | R | L |
| 3 | 8 | 10 | 12 | 13 |
| 4 | 11 | 12 | 13 | 14 |

Table 2 shows measured sizes of the macroscopic destruction zone, using spherical electrodes. As evident, the radius (R) and the length (L) almost correlates. Hence, the destruction zone is almost circular.

When using wire electrodes, the destruction zones were shaped as irregular ovals and the tissue destruction was not reproducible at the cathode. One reason for this irregular geometry is the non-uniform current distribution on the wire electrodes. The current density may be somewhat centred close to the tips of the electrode, due to the so-called edge effect, since the uptake area for the current is larger at a tip or an edge compared to a planar surface. Another reason for these irregularities may be that the wire electrodes are very thin and flabby and are therefore quite hard to apply with good precision. They may also move during the experiment as a result of muscle twitching. In the case of spherical electrodes, the electrode were easy to apply, due to the robust design, and the destruction zones were practically circular.

Claims

1. Electrode for destruction of biological tissue comprising an electrical conductor (2) with a first thickness ($t_1$), provided with an electrical insulation (3) having a second thickness ($t_2$), the conductor being connected to a current source at one end and electrically connected to an electrode head (4) at the other end of the conductor, where the electrode head serves for supplying current to the zone of the biological tissue, **characterised** in that the electrode head (4) is substantially spherical (5,10) with a diameter (D), where said diameter of the sphere is equal to or larger than said first thickness ($t_1$).

2. An electrode according to claim 1, **characterised** in that said diameter (D) of the sphere is larger than said first thickness ($t_1$).

3. An electrode according to claim 1 or 2, **characterised** in that said electrode serves as an anode.

4. An electrode according to claim 1 or 2, **characterised** in that said electrode serves as a cathode.

5. An electrode according to any of the preceding claims, **characterised** in that the current density applied with the electrode is in the range of from about $10^{-4}$ A/cm$^2$ up to about $5 \cdot 10^{-1}$ A/cm$^2$.

6. An electrode according to any of the preceding claims, **characterised** in that a constant direct current is applied.

7. An electrode according to any of the preceding claims, **characterised** in that the sphere has a diameter (D) in the range of from about 0.5 rnm up to about 3 mm, and the thickness ($t_1$) of the conductor is in the range of from about 0.2 mm up to about 2.0 mm, wherein said thickness is smaller than diameter (D) of the spherical electrode head.

8. An electrode according to any of the preceding claims, **characterised** in that at least the spherical head (4,5,10) of the electrode is made of platinum or iridium, or mixtures thereof.

9. An electrode according to any of the preceding claims, **characterised** in that the sphere is exchangeable and provided with a threaded socket.

10. An electrode according to any of the preceding claims, **characterised** in that the electrode is provided with internal channels for supplying salt solution to the area of the tissue which are to be treated.

11. An electrode according to any of the preceding claims, **characterised** in that the electrode is connected to a DC source so it serves as an anode.

12. An electrode according to any of the preceding claims, **characterised** in that the electrode is connected to a DC source to operate with a current density in the range of from about $10^{-4}$ A/cm$^2$ up to about $5 \cdot 10^{-1}$ A/cm$^2$.

Fig.1

Fig.2

Fig.3

European Patent Office

## EUROPEAN SEARCH REPORT

Application Number

EP 98 20 3216

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP 0 356 009 A (BARD INC C R) 28 February 1990 * page 4, column 5, line 17 - page 7, column 11, line 57; figures * | 1,2,5-7, 12 | A61N1/05 A61N1/40 A61N1/28 |
| A | WO 92 02272 A (SENSOR ELECTRONICS INC) 20 February 1992 * page 7, line 17 - page 14, line 30; figures * | 1,2,7 | |
| A | WO 95 10978 A (EP TECHNOLOGIES) 27 April 1995 * page 8, line 11 - page 11, line 32; figures * | 1,2,6-8 | |
| A | US 5 257 451 A (EDWARDS STUART D  ET AL) 2 November 1993 * column 2, line 41 - column 6, line 35; figures * | 1,2,7 | |
| A | EP 0 574 904 A (SIEMENS ELEMA AB) 22 December 1993 * page 3, column 3, line 38 - page 4, column 5, line 48; figures * | 1 | TECHNICAL FIELDS SEARCHED (Int.Cl.6) A61N |

The present search report has been drawn up for all claims

| Place of search | Date of completion of the search | Examiner |
|---|---|---|
| THE HAGUE | 29 January 1999 | Rakotondrajaona, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 0 911 058 A1**

## ANNEX TO THE EUROPEAN SEARCH REPORT
## ON EUROPEAN PATENT APPLICATION NO.

EP 98 20 3216

This annex lists the patent family members relating to the patent documents cited in the above-mentioned European search report.
The members are as contained in the European Patent Office EDP file on
The European Patent Office is in no way liable for these particulars which are merely given for the purpose of information.

29-01-1999

| Patent document cited in search report | | Publication date | Patent family member(s) | | Publication date |
|---|---|---|---|---|---|
| EP 0356009 | A | 28-02-1990 | US | 4896671 A | 30-01-1990 |
| | | | JP | 2099052 A | 11-04-1990 |
| WO 9202272 | A | 20-02-1992 | CA | 2070403 A | 04-02-1992 |
| | | | EP | 0497959 A | 12-08-1992 |
| | | | US | 5257635 A | 02-11-1993 |
| | | | US | 5230349 A | 27-07-1993 |
| WO 9510978 | A | 27-04-1995 | NONE | | |
| US 5257451 | A | 02-11-1993 | US | 5628775 A | 13-05-1997 |
| EP 0574904 | A | 22-12-1993 | DE | 59306009 D | 07-05-1997 |
| | | | JP | 6339533 A | 13-12-1994 |
| | | | US | 5376109 A | 27-12-1994 |

For more details about this annex : see Official Journal of the European Patent Office, No. 12/82

9